# EUROPEAN PATENT APPLICATION

(11) **EP 3 735 910 A1**
(43) Date of publication of application: **11.11.2020**
(21) Application number: 20172938.1
(22) Date of filing: 05.05.2020
(51) Int. Cl.: A61B 17/072

(54) **SURGICAL STAPLING DEVICE**

(30) Priority: 08.05.2019 US 201962844879 P; 03.04.2020 US 202016839253
(71) Applicant: Covidien LP, Mansfield, MA 02048 (US)
(72) Inventor: SCHULZ-JANDER, Daniel, Oakland, California 94602 (US)
(74) Representative: Maschio, Antonio

(57) **Abstract**

A surgical stapling device of the present disclosure includes an end effector having an anvil jaw member and a cartridge jaw member pivotally coupled to one another, the anvil jaw member and the cartridge jaw member being relatively movable such that the end effector is movable between an open position and a clamped position. A first buttress is attached to the anvil jaw member, the first buttress having at least one therapeutic or chemotherapy agent thereon, and a second buttress is attached to the cartridge jaw member.

## Description

### CROSS-REFERENCE TO RELATED APPLICATIONS

This application claims the benefit of the filing date of provisional U.S. Patent Application No. 62/844,879, filed May 8, 2019.

### TECHNICAL FIELD

The present disclosure relates to medical devices, including surgical devices such as buttresses, for use with wound closure devices. Medical devices formed of the materials of the present disclosure are capable of delivering therapeutic agents to a patient.

### BACKGROUND

Surgical stapling instruments are employed by surgeons to sequentially or simultaneously apply one or more rows of fasteners, e.g., staples or two-part fasteners, to body tissue for the purpose of joining segments of body tissue together. Such instruments generally include a pair of jaws or finger-like structures between which the body tissue to be joined is placed. When the stapling instrument is actuated, or "fired", longitudinally moving firing bars contact staple drive members in one of the jaws. The staple drive members push the surgical staples through the body tissue and into an anvil in the opposite jaw, which forms the staples. If tissue is to be removed or separated, a knife blade can be provided in the jaws of the device to cut the tissue between the lines of staples.

For some surgical procedures, it may be desirable to introduce therapeutic agents at the site of treatment.

Improved surgical repair materials, capable of use as buttresses for sealing and/or reinforcing staple lines against tissue, and improved methods for introducing therapeutic agents to a patient, remain desirable.

### SUMMARY

The present disclosure relates to medical devices, including surgical stapling devices, which can be used to repair tissue.

In embodiments, a surgical stapling device of the present disclosure includes an end effector having an anvil jaw member and a cartridge jaw member pivotally coupled to one another, the anvil jaw member and the cartridge jaw member being relatively movable such that the end effector is movable between an open position and a clamped position. A first buttress is attached to the anvil jaw member, the first buttress having at least one therapeutic agent thereon, and a second buttress is attached to the cartridge jaw member. The second buttress does not have to possess a therapeutic agent thereon. In embodiments the second buttress has at least one therapeutic agent thereon.

The therapeutic agent may be any combination of amino acids, peptides, polypeptides, proteins, polysaccharides, muteins, immunoglobulins, antibodies, cytokines, blood clotting factors, hemopoietic factors, interleukins (1 through 18), interferons, erythropoietin, nucleases, tumor necrosis factor, colony stimulating factors, insulin, anti-tumor agents and tumor suppressors, blood proteins, fibrin, thrombin, fibrinogen, synthetic thrombin, synthetic fibrin, synthetic fibrinogen, gonadotropins, hormones and hormone analogs, vaccines, somatostatin, antigens, blood coagulation factors, growth factors, bone morphogenic proteins, TGF-B, protein inhibitors, protein antagonists, protein agonists, nucleic acids, such as antisense molecules, DNA, RNA, RNAi, oligonucleotides, polynucleotides, cells, viruses, anti-inflammatory agents, anti-bacterial agents, antimicrobial agents, and ribozymes.

In some embodiments the therapeutic agent is a chemotherapy drug. Suitable chemotherapy drugs include, for example, any combination of paclitaxel and derivatives thereof, docetaxel and derivatives thereof, abraxane, tamoxifen, cyclophosphamide, actinomycin, bleomycin, dactinomycin, daunorubicin, doxorubicin, doxorubicin hydrochloride, epirubicin, mitomycin, methotrexate, fluorouracil, gemcitabine, gemcitabine hydrochloride, carboplatin, carmustine, methyl-CCNU, cisplatin, etoposide, camptothecin and derivatives thereof, phenesterine, vinblastine, vincristine, goserelin, leuprolide, interferon alfa, retinoic acid, nitrogen mustard alkylating agents, piposulfan, vinorelbine, irinotecan, irinotecan hydrochloride, vinblastine, pemetrexed, sorafenib tosylate, everolimus, erlotinib hydrochloride, sunitinib malate, capecitabine oxaliplatin, leucovorin calcium, bevacizumab, cetuximab, ramucirumab, trastuzumab, atezolizumab, canakinumab, and combinations thereof.

In embodiments, the therapeutic agent is combined with an excipient including a surfactant/solubilizer, a salt, an acid, a stabilizer, a polyhydric alcohol, a hydrotrope, a low molecular weight poly(ethylene glycol) or any combination thereof.

Suitable surfactants include cyclodextrin, sodium dodecyl sulfate, octyl glucoside, a sorbitan fatty acid ester, or combinations thereof.

In embodiments, suitable salts include sodium chloride.

In some embodiments, the acid includes oleic acid, citric acid, ascorbic acid, or combinations thereof.

In other embodiments, the stabilizer includes butylated hydroxytoluene, or butylated hydroxyanisole.

In yet other embodiments, the polyhydric alcohol includes D-sorbitol, mannitol, or combinations thereof.

In some embodiments, the first buttress is attached to the anvil jaw member by at least one suture.

In other embodiments, the second buttress is attached to the cartridge jaw member by at least one suture.

In other embodiments, a surgical stapling device of the present disclosure includes an end effector including an anvil jaw member and a cartridge jaw member pivotally coupled to one another, the anvil jaw member and the cartridge jaw member being relatively movable such that the end effector is movable between an open position and a clamped position. The surgical stapling device includes a first buttress attached to the anvil jaw member, the first buttress having at least one chemotherapy drug thereon, and a second buttress attached to the cartridge jaw member. The second buttress does not have to possess a chemotherapy drug thereon. In embodiments, the second buttress has at least one chemotherapy drug thereon.

Methods for stapling tissue with the stapling device of the present disclosure are also provided.

### BRIEF DESCRIPTION OF THE DRAWINGS

Embodiments of the presently disclosed surgical stapling apparatus are described herein with reference to the drawings wherein:
FIG. 1 is a perspective view of a surgical stapling apparatus including a handle housing, an adapter assembly, an end effector, and buttresses attached to components thereof in accordance with an embodiment of the present disclosure;
FIG. 2 is an enlarged view of the indicated area of detail shown in FIG. 1;
FIG. 3 is a perspective view of an anvil assembly of the end effector of the surgical stapling apparatus shown in FIG. 1, showing how a buttress in accordance with an embodiment of the present disclosure may be attached thereto;
FIG. 4 is a perspective view of a cartridge assembly of the end effector of the surgical stapling apparatus shown in FIG. 1, showing how a buttress in accordance with an embodiment of the present disclosure may be attached thereto;
FIG. 5 is a cross-sectional view taken along section line 5-5 of FIG. 2, showing the configuration of the buttresses of the present disclosure attached to the anvil assembly and the cartridge assembly, prior to firing of the surgical stapling apparatus of FIG. 1;
FIG. 6 is an enlarged view of the indicated area of detail shown in FIG. 5;
FIG. 7 is a perspective view of a stapled and divided section of tissue after firing of the surgical stapling apparatus of FIG. 1; and
FIG. 8 is a side view of the stapled and divided section of tissue of FIG. 7, after firing of the surgical stapling apparatus of FIG. 1.

### DETAILED DESCRIPTION

Various exemplary embodiments of the present disclosure are discussed herein below in terms of buttresses for use with tissue fixation devices, in embodiments surgical staples. While the below disclosure discusses in detail the use of these buttresses with staples, it will be appreciated that surgical stapling apparatuses of the present disclosure include a range of buttressing materials and film-based materials that may be used to mechanically support tissues, reinforce tissues along staple or suture lines, and decrease the incidence of fluid leakage and/or bleeding of tissues. For example, other suitable materials which may be used with the surgical stapling apparatus of the present disclosure include hernia patches and/or tissue scaffolds.

The buttress used with a surgical stapling apparatus of the present disclosure is in the form of a generally rectangular body having a distal end and a proximal end, with opposing lateral sides that run along the length of the elongate rectangular body portion from the distal end to the proximal end.

In embodiments, a buttress of the present disclosure may possess a therapeutic agent. The material used to form the buttress may be impregnated with the therapeutic agent, the therapeutic agent may be applied to the buttress as a coating, or combinations of this application of therapeutic agent(s) may be used. In some embodiments, as set forth in greater detail below, additional layers and/or coatings may be applied to buttresses of the present disclosure.

Therapeutic agents added to the buttress of the present disclosure are suitable for further treatment of tissue at or near the site where the surgical buttress of the present disclosure is placed. Thus, the present disclosure describes surgical buttresses, and methods and mechanisms for using the same, for the targeted delivery of therapeutic agents to a patient.

It should be understood that a variety of surgical stapling apparatuses may be utilized with a surgical buttress of the present disclosure. In embodiments, linear staplers may be utilized such as, for example, those including EndoGIA™ Reinforced Reload with Tri-Staple Technology™ and other staplers with Tri-Staple™ technology, available through Medtronic, (North Haven, CT), as well as other anastomosis staplers, such as, for example, EEA™, CEEA™, GIA™, EndoGIA™, and TA™, also available through Medtronic. It should also be appreciated that the principles of the present disclosure are equally applicable to surgical staplers having alternate configurations, such as, for example, end-to-end anastomosis staplers having a circular cartridge and anvil (see, e.g., commonly owned U.S. Patent No. 5,915,616, entitled "Surgical Fastener Applying Apparatus," the entire disclosure of which is incorporated by reference herein); laparoscopic staplers (see, e.g., commonly owned U.S. Pat. Nos. 6,330,965 and 6,241,139, each entitled "Surgical Stapling Apparatus," the entire disclosures of each of which are incorporated by reference herein); and transverse anastomosis staplers (see, e.g., commonly owned U.S. Patent Nos. 5,964,394 and 7,334,717, each entitled "Surgical Fastener Applying Apparatus", the entire disclosures of each of which are incorporated by reference herein).

Embodiments of the presently disclosed surgical buttress and surgical stapling apparatus will now be described in detail with reference to the figures wherein like reference numerals identify similar or identical elements. In the following discussion, the terms "proximal" and "trailing" may be employed interchangeably, and should be understood as referring to the portion of a structure that is closer to a clinician during proper use. The terms "distal" and "leading" may also be employed interchangeably, and should be understood as referring to the portion of a structure that is further from the clinician during proper use. As used herein, the term "patient" should be understood as referring to a human subject or other animal, and the term "clinician" should be understood as referring to a doctor, nurse, or other care provider and may include support personnel.

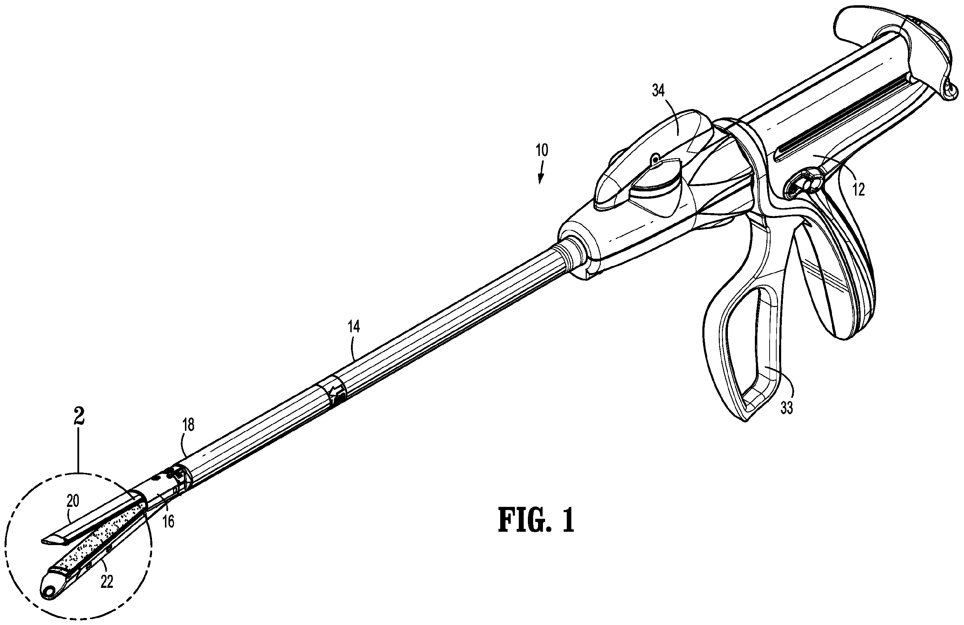

Referring now to FIG. 1, there is disclosed an exemplary surgical stapling apparatus or surgical stapler 10 for use in stapling tissue and applying a surgical buttress to tissue. The surgical stapling apparatus 10 generally includes a handle 12 having an elongate tubular member 14 extending distally from the handle 12. An end effector 16 is mounted on a distal end 18 of the elongate tubular member 14. The end effector 16 includes an anvil assembly including a staple clinching anvil jaw member 20 and a cartridge assembly including a staple cartridge jaw member 22 configured to receive a staple cartridge 32. The end effector 16 may be permanently affixed to the elongate tubular member 14 or may be detachable and thus replaceable with a new end effector 16. The staple clinching anvil jaw member 20 is movably mounted on the distal end 18 of the end effector 16 and is movable between an open position spaced apart from the staple cartridge jaw member 22 to a closed position substantially adjacent the staple cartridge jaw member 22.

The surgical stapling apparatus 10 further includes a trigger 33, as seen in FIG. 1, movably mounted on the handle 12. Actuation of the trigger 33 initially operates to move the anvil jaw member 20 from the open to the closed position relative to the staple cartridge jaw member 22 and subsequently actuates the surgical stapling apparatus 10 to apply lines of staples to tissue. In order to properly orient the end effector 16 relative to the tissue to be stapled, the surgical stapling apparatus 10 is additionally provided with a rotation knob 34 mounted on the handle 12. Rotation of the rotation knob 34 relative to the handle 12 rotates the elongate tubular member 14 and the end effector 16 relative to the handle 12 so as to properly orient the end effector 16 relative to the tissue to be stapled.

Reference may be made to commonly owned U.S. Patent Nos. 5,915,616, 6,330,965, and 6,241,139, the disclosures of each of which are incorporated by reference herein, for a detailed discussion of the construction and operation of the surgical stapling apparatus 10.

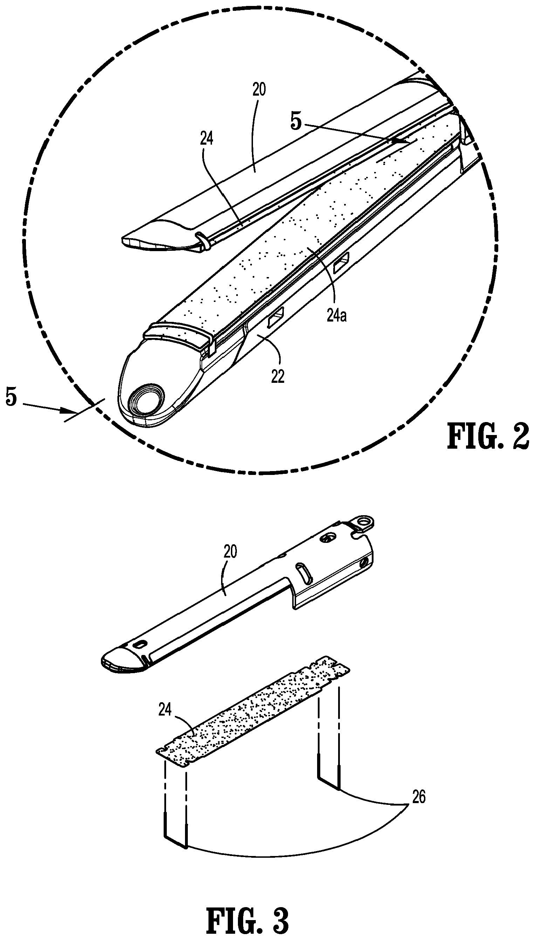

Referring to FIGS. 2-4, the staple clinching anvil jaw member 20 and the staple cartridge jaw member 22 may be provided with surgical buttresses 24, 24a. The surgical buttresses 24, 24a are provided to reinforce and seal staple lines applied to tissue by the surgical stapling apparatus 10. The surgical buttresses 24, 24a may be configured in any shape, size, or dimension suitable to fit any surgical stapling, fastening, or firing apparatus.

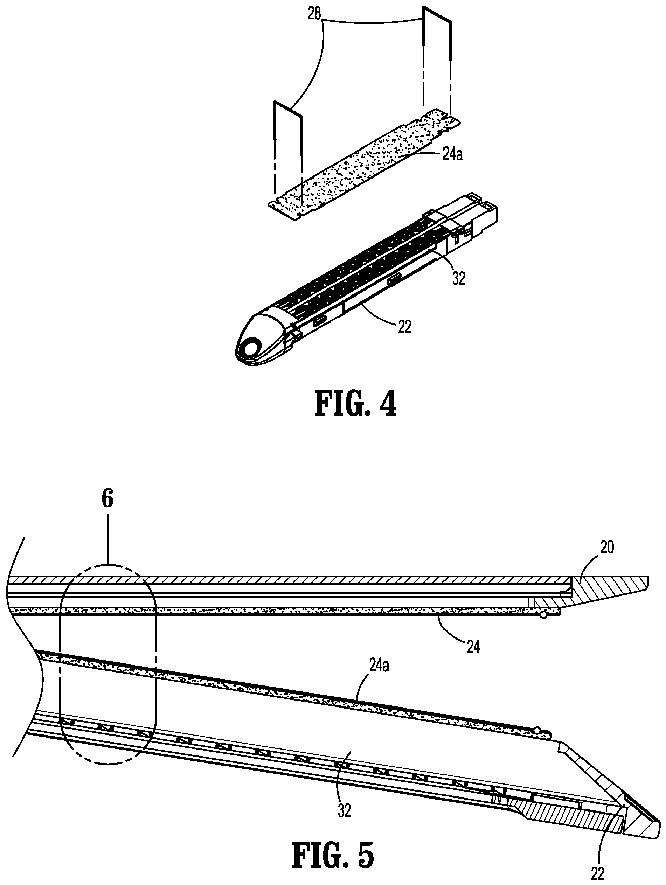

As illustrated in FIG. 3, the surgical buttress 24 may be attached to the staple clinching anvil jaw member 20 with sutures 26. Similarly, as illustrated in FIG. 4, the surgical buttress 24a may be attached to the staple cartridge jaw member 22 with sutures 28.

As illustrated in FIGS. 3-5, the staple clinching anvil jaw member 20 has been loaded with a surgical buttress 24, and the staple cartridge jaw member 22, including the staple cartridge 32, has been loaded with a surgical buttress 24a.

Surgical buttresses in accordance with the present disclosure may be fabricated from a biocompatible substrate material. Such substrates may be formed of bioabsorbable, non-absorbable, natural and/or synthetic materials.

In embodiments, the surgical buttress may be biodegradable, so that the surgical buttress does not have to be retrieved from the body. The term "biodegradable" as used herein is defined to include both bioabsorbable and bioresorbable materials. By biodegradable, it is meant that the surgical buttress decomposes or loses structural integrity under body conditions (e.g., enzymatic degradation or hydrolysis), or is broken down (physically or chemically) under physiologic conditions in the body such that the degradation products are excretable or absorbable by the body.

Non-limiting examples of materials which may be used in forming a surgical buttress of the present disclosure include, but are not limited to, poly(lactic acid), poly(glycolic acid), poly(trimethylene carbonate), poly(dioxanone), poly(hydroxybutyrate), poly(phosphazine), polyethylene terephthalate, polyethylene glycols, polyethylene oxides, polyacrylamides, polyhydroxyethylmethylacrylate, polyvinylpyrrolidone, polyvinyl alcohols, polyacrylic acid, polyacetate, polycaprolactone, polypropylene, aliphatic polyesters, glycerols, poly(amino acids), copoly(ether-esters), polyalkylene oxalates, polyamides, poly(iminocarbonates), polyalkylene oxalates, polyoxaesters, polyorthoesters, polyphosphazenes, and copolymers, block copolymers, homopolymers, blends and combinations thereof.

In embodiments, natural biological polymers may be used in forming a surgical buttress of the present disclosure. Suitable natural biological polymers include, but are not limited to, collagen, gelatin, fibrin, fibrinogen, elastin, keratin, albumin, cellulose, oxidized cellulose, hydroxyethyl cellulose, hydroxypropyl cellulose, carboxyethyl cellulose, carboxymethyl cellulose, chitin, chitosan, and combinations thereof. In addition, natural biological polymers may be combined with any of the other polymeric materials described herein to produce a surgical buttress of the present disclosure.

The surgical buttress may also be formed of materials that are porous or non-porous. It should of course be understood that any combination of porous, non-porous, natural, synthetic, bioabsorbable, and/or non-bioabsorbable materials may be used to form a surgical buttress of the present disclosure.

In some embodiments, a surgical buttress of the present disclosure may be formed of porous material(s). Any porous portion of a surgical buttress of the present disclosure may have openings or pores over at least a part of a surface thereof. Suitable porous materials include, but are not limited to, fibrous structures (e.g., knitted structures, woven structures, non-woven structures, etc.) and/or foams (e.g., open or closed cell foams).

In embodiments, the pores may be in sufficient number and size so as to interconnect across the entire thickness of the surgical buttress. Woven fabrics, knitted fabrics, non-woven fabrics and open cell foams are illustrative examples of structures in which the pores can be in sufficient number and size so as to interconnect across the entire thickness of the surgical buttress.

In other embodiments, the pores may not interconnect across the entire thickness of the surgical buttress. Closed cell foams or fused non-woven materials are illustrative examples of structures in which the pores may not interconnect across the entire thickness of the surgical buttress. In some embodiments, pores may be located on a portion of the surgical buttress, with other portions of the surgical buttress having a non-porous texture. Those skilled in the art may envision a variety of pore distribution patterns and configurations for a porous surgical buttress of the present disclosure.

Where the surgical buttress of the present disclosure is porous and includes fibrous materials, the surgical buttress may be formed using any suitable method including, but not limited to, knitting, weaving, non-woven techniques (including melt blowing), wet-spinning, electro-spinning, extrusion, co-extrusion, and the like. In embodiments, the surgical buttress possesses a three dimensional structure, such as the textiles described in U.S. Patent Nos. 7,021,086 and 6,443,964, the entire disclosures of each of which are incorporated by reference herein.

The porosity of the fabric used to form the substrate may allow for the infiltration of biological fluids and/or cellular components which, in turn, may accelerate the release kinetics of any therapeutic agent from the medical device of the present disclosure, thus increasing the rate of release of therapeutic agent(s) from the surgical buttress into the surrounding tissue and fluids.

Substrates used to form surgical buttresses of the present disclosure may have a thickness from about 0.05 mm to about 0.5 mm, in embodiments from about 0.1 mm to about 0.2 mm.

Where the substrate used to form the surgical buttress is porous, the surgical buttress of the present disclosure may have a pore volume from about 65% to about 85%, in embodiments from about 70% to about 80%.

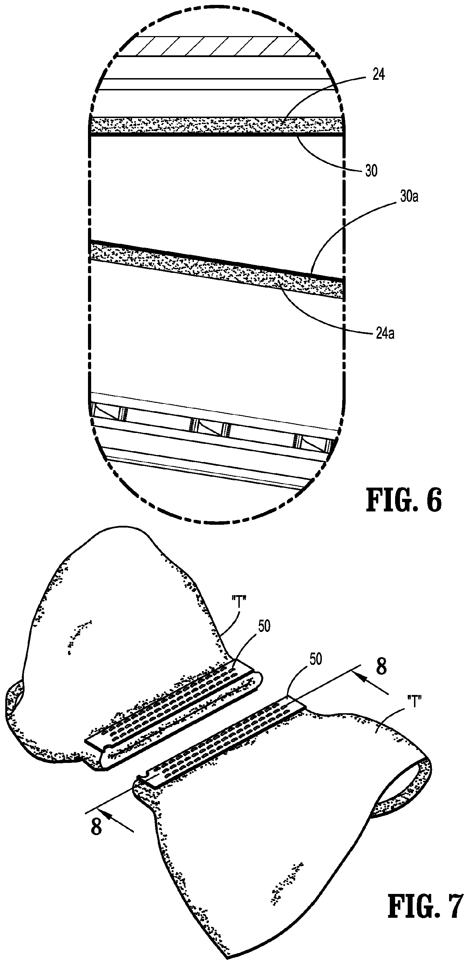

As depicted in FIG. 6, in embodiments the buttresses 24, 24a have a therapeutic agent therein. In addition, FIG. 6 depicts the buttresses 24, 24a having an outer layer 30, 30a thereon, respectively.

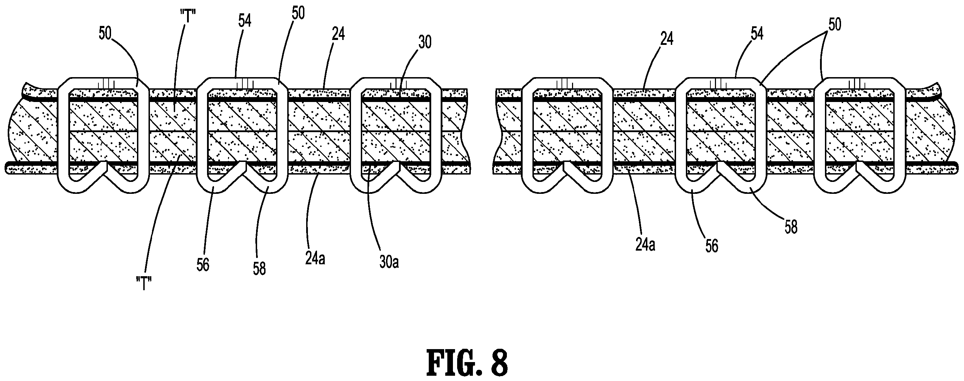

In use, as generally depicted in FIGS. 7-8, the buttresses 24, 24a described herein may be used in sealing a wound by approximating the edges of wound tissue between the staple cartridge jaw member 22 and the anvil jaw member 20 of the surgical stapling apparatus 10. Firing of the surgical stapling apparatus 10 forces the staple legs 56, 58 of at least one staple 50 to pass through the openings on the staple cartridge jaw member 22, the buttress 24a on the staple cartridge jaw member 22, the tissue "T", the buttress 24 on the anvil jaw member 20, and the openings on the anvil (not shown) to secure the buttresses 24, 24a to the tissue "T" so that the tissue is sandwiched between the two, thereby securing the adjoining tissue and to seal the tissue.

The resulting tissue "T", divided and stapled closed with staples 50, is illustrated in FIGS. 7-8. Specifically, the surgical buttress 24a that was associated with the staple cartridge jaw member 22 is secured against tissue "T" by backspans 54 of staples 50 and the surgical buttress 24 associated with the anvil jaw member 20 is secured against tissue "T" by staple legs 56 and 58. Thus, surgical buttresses 24, 24a are stapled to tissue "T" thereby sealing and reinforcing the staple lines created by staples 50.

While the above description is directed to rectangular buttresses, it is to be appreciated that any suitable configuration for a buttress may be utilized in accordance with the present disclosure. For example, buttresses having an elongate rectangular body with head and tail portions at the ends of the buttress may be utilized. Any other suitable shape may be utilized. For example, additional suitable buttresses include those disclosed in U.S. Patent Application Serial No. 15/639,367, filed June 30, 2017, and U.S. Patent Nos. 8,157,151, 8,561,873 and 9,693,772, the entire disclosures of each of which are incorporated by reference herein.

Therapeutic agents which may be added to a surgical buttress include, but are not limited to, drugs, amino acids, peptides, polypeptides, proteins, polysaccharides, muteins, immunoglobulins, antibodies, cytokines (e.g., lymphokines, monokines, chemokines), blood clotting factors, hemopoietic factors, interleukins (1 through 18), interferons (β-IFN, a-IFN and γ-IFN), erythropoietin, nucleases, tumor necrosis factor, colony stimulating factors (e.g., GCSF, GM-CSF, MCSF), insulin, anti-tumor agents and tumor suppressors, blood proteins, fibrin, thrombin, fibrinogen, synthetic thrombin, synthetic fibrin, synthetic fibrinogen, gonadotropins (e.g., FSH, LH, CG, etc.), hormones and hormone analogs (e.g., growth hormone, luteinizing hormone releasing factor), vaccines (e.g., tumoral, bacterial and viral antigens), somatostatin, antigens, blood coagulation factors, growth factors (e.g., nerve growth factor, insulin-like growth factor), bone morphogenic proteins, TGF-B, protein inhibitors, protein antagonists, protein agonists, nucleic acids, such as antisense molecules, DNA, RNA, RNAi, oligonucleotides, polynucleotides, cells, viruses, anti-inflammatory agents, anti-bacterial agents, antimicrobial agents, and ribozymes.

In embodiments, the therapeutic agent applied to a surgical buttress of the present disclosure may include an anti-tumor agent and/or tumor suppressor, referred to, in embodiments, as a "chemotherapeutic agent" and/or an "antineoplastic agent." Suitable chemotherapeutic agents include, for example, paclitaxel and derivatives thereof, docetaxel and derivatives thereof, abraxane, tamoxifen, cyclophosphamide, actinomycin, bleomycin, dactinomycin, daunorubicin, doxorubicin, doxorubicin hydrochloride, epirubicin, mitomycin, methotrexate, fluorouracil, gemcitabine, gemcitabine hydrochloride, carboplatin, carmustine (BCNU), methyl-CCNU, cisplatin, etoposide, camptothecin and derivatives thereof, phenesterine, vinblastine, vincristine, goserelin, leuprolide, interferon alfa, retinoic acid (ATRA), nitrogen mustard alkylating agents, piposulfan, vinorelbine, irinotecan, irinotecan hydrochloride, vinblastine, pemetrexed, sorafenib tosylate, everolimus, erlotinib hydrochloride, sunitinib malate, capecitabine oxaliplatin, leucovorin calcium, bevacizumab, cetuximab, ramucirumab, trastuzumab, atezolizumab, canakinumab, combinations thereof, and the like.

In embodiments, paclitaxel and/or paclitaxel derivatives may be used as the therapeutic agent. Paclitaxel may have various forms, referred to herein as "polymorphs," including amorphous paclitaxel, crystalline paclitaxel, sometimes referred to as crystalline paclitaxel dihydrate, and/or anhydrous paclitaxel, or mixtures thereof.

In accordance with the present disclosure, the polymorph form of paclitaxel utilized in forming the therapeutic layer may be varied by the aqueous composition, the solvent polarity and the composition of protic and aprotic solvents utilized in the solvent system to form the solution for applying the therapeutic layer. For example, paclitaxel dissolved and then dried from 10% v/v water in methanol will yield a predominantly crystalline paclitaxel dihydrate layer, while the same paclitaxel dissolved and then dried from non-polar solvent dichloromethane will yield a predominantly amorphous layer.

The crystallinity of the paclitaxel will impact its solubility in aqueous systems. Accordingly, the polymorph mixture of paclitaxel in the therapeutic layer may be adjusted and selected to provide a tailored release of therapeutic agent from the surgical buttress of the present disclosure. Although the drug in any form is hydrophobic, amorphous paclitaxel is more soluble in aqueous environments, and crystalline paclitaxel is less soluble in aqueous environments. Accordingly, in embodiments, more than one polymorphic form of paclitaxel may be used to provide implants that have multiple release profiles of paclitaxel. For example, surgical buttresses of the present disclosure having both amorphous paclitaxel and crystalline paclitaxel (dihydrate or anhydrous) thereon may release a bolus of therapeutic agent upon implantation (resulting primarily by amorphous paclitaxel dissolution), while also slowly releasing the therapeutic agent (resulting primarily by crystalline paclitaxel dissolution).

In embodiments with no excipient, the amount of amorphous paclitaxel in the therapeutic layer on the surgical buttress may be from 0 % to about 100 % by weight of the therapeutic layer, in embodiments from about 10 % to about 90 % by weight of the therapeutic layer, with the crystalline paclitaxel being present in amounts from about 0 to about 100% by weight of the therapeutic layer, in embodiments from about 90 % to about 10 % by weight of the therapeutic layer.

Surgical buttresses of the present disclosure may release amorphous paclitaxel in vivo over a period of time from about 18 hours to about 96 hours, in embodiments from about 24 hours to about 72 hours, and release the crystalline paclitaxel in vivo over a period of time from about 3 days to about 14 days, in embodiments from about 7 days to about 10 days.

In some embodiments, the therapeutic layer forming a coating along at least a portion of the buttress may be formed of polymeric materials or other carrier components within the purview of those skilled in the art. In embodiments, such layers may include, for example, degradable materials such as those prepared from monomers such as glycolide, lactide, trimethylene carbonate, p-dioxanone, epsilon-caprolactone, and combinations thereof.

In other embodiments, regardless of whether the therapeutic agent is applied with or without some additional polymeric material to form the therapeutic layer, in addition to the therapeutic agents described above, therapeutic layers applied to the substrate material in forming a surgical buttress of the present disclosure may also include excipients to enhance both the ability of the therapeutic agent to adhere to the surgical buttress, as well as to modify the elution of the therapeutic agent from the surgical buttress.

In embodiments, suitable excipients which may be combined with a therapeutic agent to form the therapeutic layer on the surgical buttress include surfactants such as, but not limited to, cyclodextrins such as 2-hydroxypropyl-beta-cyclodextrin and methyl-B-cyclodextrin, sodium dodecyl sulfate, octyl glucoside, and sorbitan fatty acid esters such as sorbitan monooleate, sorbitan monolaurate and polyethoxylated fatty acid esters of sorbitan, sometimes referred to herein as polysorbates, including those sold under the name TWEEN™. Examples of such polysorbates include polysorbate 80 (TWEEN™ 80), polysorbate 20 (TWEEN™ 20), polysorbate 60 (TWEEN™ 60), polysorbate 65 (TWEEN™ 65), polysorbate 85 (TWEEN™ 85), combinations thereof, and the like. In embodiments, low molecular weight poly(ethylene glycol)s may be added as an excipient, either alone or in any combination with any of the other above excipients.

In other embodiments, suitable excipients may include salts such as sodium chloride and/or other materials such as urea, oleic acid, citric acid, and ascorbic acid. In yet other embodiments, the excipient may be a stabilizer such as butylated hydroxytoluene (BHT) or butylated hydroxyanisole (BHA).

Still other suitable excipients include polyhydric alcohols such as D-sorbitol, mannitol, combinations thereof, and the like.

In some embodiments, excipients which are hydrotropes may be included in the therapeutic layers of the present disclosure. These materials attract water into the therapeutic layer, which may enhance its degradation and resulting release of the therapeutic agent from the therapeutic layer.

In embodiments, the therapeutic agent(s), carrier component(s) and/or excipient(s) may be in a solution for application to a surgical buttress of the present disclosure. Any suitablesolvent that can be removed by drying may be used to form such a solution. Suitable solvents for forming such a solution include any pharmaceutically acceptable solvents including, but not limited to, saline, water, alcohol, acetone, dimethyl sulfoxide, ethyl acetate, N-methylpyrrolidone, combinations thereof, and the like. Methods for forming such solutions are within the purview of those skilled in the art and include, but are not limited to, mixing, blending, sonication, heating, combinations thereof, and the like.

In embodiments, by selecting different solvent systems, different dissolution rates of the therapeutic agent(s) may be achieved due to different therapeutic agent morphologies and degrees of crystallinity that occur based upon the solvent used in forming the solution including the therapeutic agent(s).

In embodiments, the therapeutic agent(s), any carrier component(s), and/or any excipient(s) may be applied to the surgical buttress by a needle deposition process. As noted above, in embodiments the therapeutic agent is in a solution, which is then applied to a surgical buttress.

In embodiments, the therapeutic agent(s), any carrier component(s), and/or any excipient(s) may be applied to the medical device of the present disclosure prior to affixing the medical device to some other medical apparatus. For example, in the case of a buttress, the buttress may be coated in accordance with the present disclosure prior to its attachment to a surgical stapler.

In embodiments, additional outer layers may be applied over a therapeutic agent coating layer on a surgical buttress of the present disclosure. Such additional layers, in embodiments, may be non-permeable, semi-permeable, or porous, to permit adjustment of the rate of release of a therapeutic agent from the therapeutic agent coating layer on a surgical buttress of the present disclosure.

Suitable materials for forming an outer layer on a surgical buttress include, for example, degradable materials such as those prepared from monomers such as glycolide, lactide, trimethylene carbonate, p-dioxanone, epsilon-caprolactone, and combinations thereof. In embodiments, suitable materials for forming an outer layer on a surgical buttress include, for example, phosphorylcholine polymers.

In accordance with the present disclosure, the therapeutic layer, including the therapeutic agent(s), solvent(s), any carrier component(s), and/or any excipient(s), is applied so that an adequate amount of therapeutic agent(s) is deposited and stays robustly attached to the surgical buttress.

After application, the solvent from the coating solution may be driven off by methods within the purview of those skilled in the art. For example, solvent evaporation may be facilitated by heat, gas flow, time, reduced pressure, combinations thereof, and the like, to increase the accuracy of drug deposition on the surgical buttress. Moreover, this assisted evaporation of solvent may be applied to the whole surface of the surgical buttress, partially to only a portion of the surface of the surgical buttress, or just around the deposition instrument (e.g., a needle tip).

Driving off the solvent leaves the therapeutic agent and any carrier component and/or excipient behind to form the therapeutic layer on the surgical buttress.

In accordance with the present disclosure, the process may be repeated, such that multiple passes can be made so that the surface of the surgical buttress has the desired amount of therapeutic agent for administering a dose of the therapeutic agent. In embodiments, repeating the process described above results in the deposition of multiple layers such that the overall therapeutic layer on the buttress is very uniform and robust, and adheres to the buttress material very well. This is in contrast to other processes, such as dip coating and other similar coating methods, which lack both the robustness and adherence of the coatings/layers produced in accordance with the present disclosure.

The process is designed in such a way that it leverages the capillary action of the fabric on which the drug is dispensed for coating. The speed and rate of dispersion are appropriately controlled to produce the desired coatings. Between coatings, the process may have pre-determined pauses to ensure each coat has the proper time to dry before more therapeutic agent is deposited as a next layer.

Utilizing the processes of the present disclosure, there is limited drug loss during the different stages of the process. This is beneficial in terms of isolating the therapeutic agent to areas where it is intended to stay, and cost savings in terms of the amount of therapeutic agent being used.

In embodiments, one can coat directly on the buttress rather than coat on cartridge and anvil assemblies, which saves with respect to material handling, labor costs and quality related costs. Moreover, it is much safer for the operators. For example, the process is developed such that it is better than spray coating which can be very hazardous for operators running the process. A normal air flow hood would suffice for operating this process safely. Also, an isolator is not needed for this process which makes it very ergonomic for long term manufacturing.

Utilizing the methods of the present disclosure, a therapeutic agent may be deposited on specific areas on the device with high precision. Certain sections of the surface of the buttress can be left non-coated by design to improve the performance of the surgical buttress, for instance better tissue healing around the staple line. In addition, varying amounts of therapeutic agent(s) may be applied to different areas of the surgical buttress, thereby creating concentration gradients of the therapeutic agent(s) on the surgical buttress and/or focused application of therapeutic agent(s) from selected portions of the surgical buttress.

In embodiments, multiple layers of therapeutic agents can be deposited on the surgical buttress. In some cases, different therapeutic agents are applied in different layers. Different therapeutic benefits can thus be combined on one device by using the multiple layers. In other embodiments, different therapeutic agents can be deposited on different areas on the surface of the surgical buttress, e.g., one therapeutic agent can be applied in one region/area, and a different therapeutic agent can be applied to a different region/area.

After formation, surgical buttress of the present disclosure may possess the therapeutic agent in the coated buttress thereon in amounts from about 0.1 % to about 50 % by weight of the coated buttress, in embodiments from about 1 % to about 10 % by weight of the coated buttress. While excipients are not required, where present, non-polymeric excipients may be present in an amount from about 0.01 % to about 80% by weight of the coated buttress, in embodiments from about 1 % to about 11 % by weight of the coated buttress. In other embodiments, where present, polymeric excipients may be present in an amount from about 0.014% to about 14% by weight of the coated buttress, in embodiments from about 5% to about 15% by weight of the coated buttress.

After formation, surgical buttresses of the present disclosure may possess the therapeutic agent in the therapeutic layer thereon in amounts from about 0.01 % to about 100 % by weight of the therapeutic layer, in embodiments from about 1% to about 75 % by weight of the therapeutic layer. While excipients are not required, where present, non-polymeric excipients may be present in an amount from about 1% to about 99% by weight of the therapeutic layer, in embodiments from about 8.5% to about 79.4% by weight of the therapeutic layer, in other embodiments from 9.5% to about 15%. In embodiments, where present, polymeric excipients may be present in an amount from about 1% to about 99% by weight of the therapeutic layer, in embodiments from about 5% to about 15% by weight of the therapeutic layer.

A therapeutic layer having both a therapeutic agent and non-polymeric excipients may have a thickness from about 13 nm to about 2.9 µm, in embodiments from about 25 nm to about 100 nm.

A therapeutic layer having both a therapeutic agent and polymeric excipients may have a thickness from about 2 nm to about 1.1 µm, in embodiments from about 30 nm to about 100 nm.

In other embodiments, the therapeutic layers may include little or no excipients, so very thin therapeutic layers may be applied to the substrate. This will maintain the porosity of the substrate. Such therapeutic layers may have a thickness from about 11 nm to about 218 nm, in embodiments from about 25 nm to about 75 nm.

Surgical buttresses of the present disclosure may release therapeutic agents therefrom over a period of time from about 18 hours to about 4 weeks, in embodiments from about 48 hours to about 2 weeks.

In embodiments, as noted above, a surgical buttress of the present disclosure is provided to reinforce and seal the lines of staples applied to tissue by a surgical stapling apparatus. Upon application to a site of bleeding tissue, the surgical buttress may affect hemostasis of said tissue. As used herein, the term "hemostasis" means the arrest of bleeding.

In addition to providing hemostasis at the site of application of the surgical buttress, the surgical buttresses of the present disclosure may also provide for treatment of tissue with the therapeutic agent at both the site of implantation and elsewhere in the body.

It will be understood that various modifications may be made to the embodiments disclosed herein. Therefore, the above description should not be construed as limiting, but merely as an exemplification of preferred embodiments. Those skilled in the art will envision other modifications within the scope and spirit of the present disclosure. Such modifications and variations are intended to come within the scope of the following claims.

The invention may be described by reference to the following numbered paragraphs:
1. A surgical stapling device, comprising: an end effector including an anvil jaw member and a cartridge jaw member pivotally coupled to one another, the anvil jaw member and the cartridge jaw member being relatively movable such that the end effector is movable between an open position and a clamped position; a first buttress attached to the anvil jaw member, the first buttress having at least one therapeutic agent thereon; and a second buttress attached to the cartridge jaw member.
2. The surgical stapling device of paragraph 1, wherein the second buttress has at least one therapeutic agent thereon.
3. The surgical stapling device of paragraph 1, wherein the therapeutic agent is any combination of amino acids, peptides, polypeptides, proteins, polysaccharides, muteins, immunoglobulins, antibodies, cytokines, blood clotting factors, hemopoietic factors, interleukins (1 through 18), interferons, erythropoietin, nucleases, tumor necrosis factor, colony stimulating factors, insulin, anti-tumor agents and tumor suppressors, blood proteins, fibrin, thrombin, fibrinogen, synthetic thrombin, synthetic fibrin, synthetic fibrinogen, gonadotropins, hormones and hormone analogs, vaccines, somatostatin, antigens, blood coagulation factors, growth factors, bone morphogenic proteins, TGF-B, protein inhibitors, protein antagonists, protein agonists, nucleic acids, such as antisense molecules, DNA, RNA, RNAi, oligonucleotides, polynucleotides, cells, viruses, anti-inflammatory agents, anti-bacterial agents, antimicrobial agents, and ribozymes.
4. The surgical stapling device of paragraph 1, wherein the therapeutic agent is a chemotherapy drug.
5. The surgical stapling device of paragraph 4, wherein the chemotherapy drug is any combination of paclitaxel and derivatives thereof, docetaxel and derivatives thereof, abraxane, tamoxifen, cyclophosphamide, actinomycin, bleomycin, dactinomycin, daunorubicin, doxorubicin, doxorubicin hydrochloride, epirubicin, mitomycin, methotrexate, fluorouracil, gemcitabine, gemcitabine hydrochloride, carboplatin, carmustine, methyl-CCNU, cisplatin, etoposide, camptothecin and derivatives thereof, phenesterine, vinblastine, vincristine, goserelin, leuprolide, interferon alfa, retinoic acid, nitrogen mustard alkylating agents, piposulfan, vinorelbine, irinotecan, irinotecan hydrochloride, vinblastine, pemetrexed, sorafenib tosylate, everolimus, erlotinib hydrochloride, sunitinib malate, capecitabine oxaliplatin, leucovorin calcium, bevacizumab, cetuximab, ramucirumab, trastuzumab, atezolizumab, canakinumab, and combinations thereof.
6. The surgical stapling device of paragraph 1, wherein the therapeutic agent is combined with an excipient including a surfactant, a salt, an acid, a stabilizer, a polyhydric alcohol, a hydrotrope, a low molecular weight poly(ethylene glycol) or any combination thereof.
7. The surgical stapling device of paragraph 6, wherein the surfactant is a cyclodextrin, sodium dodecyl sulfate, octyl glucoside, a sorbitan fatty acid ester, or combinations thereof.
8. The surgical stapling device of paragraph 6, wherein the salt includes sodium chloride.
9. The surgical stapling device of paragraph 6, wherein the acid includes oleic acid, citric acid, ascorbic acid, or combinations thereof.
10. The surgical stapling device of paragraph 6, wherein the stabilizer includes butylated hydroxytoluene, or butylated hydroxyanisole.
11. The surgical stapling device of paragraph 6, wherein the polyhydric alcohol includes D-sorbitol, mannitol, or combinations thereof.
12. The surgical stapling device of paragraph 1, wherein the first buttress is attached to the anvil jaw member by at least one suture.
13. The surgical stapling device of paragraph 1, wherein the second buttress is attached to the cartridge jaw member by at least one suture.
14. A method for treating tissue comprising stapling tissue with the surgical stapling device of paragraph 1.
15. A surgical stapling device, comprising: an end effector including an anvil jaw member and a cartridge jaw member pivotally coupled to one another, the anvil jaw member and the cartridge jaw member being relatively movable such that the end effector is movable between an open position and a clamped position; a first buttress attached to the anvil jaw member, the first buttress having at least one chemotherapy drug thereon; and a second buttress attached to the cartridge jaw member.
16. The surgical stapling device of paragraph 15, wherein the second buttress has at least one chemotherapy drug thereon.
17. The surgical stapling device of paragraph 15, wherein the chemotherapy drug is any combination of paclitaxel and derivatives thereof, docetaxel and derivatives thereof, abraxane, tamoxifen, cyclophosphamide, actinomycin, bleomycin, dactinomycin, daunorubicin, doxorubicin, doxorubicin hydrochloride, epirubicin, mitomycin, methotrexate, fluorouracil, gemcitabine, gemcitabine hydrochloride, carboplatin, carmustine, methyl-CCNU, cisplatin, etoposide, camptothecin and derivatives thereof, phenesterine, vinblastine, vincristine, goserelin, leuprolide, interferon alfa, retinoic acid, nitrogen mustard alkylating agents, piposulfan, vinorelbine, irinotecan, irinotecan hydrochloride, vinblastine, pemetrexed, sorafenib tosylate, everolimus, erlotinib hydrochloride, sunitinib malate, capecitabine oxaliplatin, leucovorin calcium, bevacizumab, cetuximab, ramucirumab, trastuzumab, atezolizumab, canakinumab, and combinations thereof.
18. The surgical stapling device of paragraph 17, wherein the chemotherapy drug is combined with an excipient including a surfactant, a salt, an acid, a stabilizer, a polyhydric alcohol, a hydrotrope, a low molecular weight poly(ethylene glycol) or any combination thereof.
19. The surgical stapling device of paragraph 15, wherein the first buttress is attached to the anvil jaw member by at least one suture.
20. The surgical stapling device of paragraph 15, wherein the second buttress is attached to the cartridge jaw member by at least one suture.
21. A method for treating tissue comprising stapling tissue with the surgical stapling device of paragraph 15.

## Claims

1. A surgical stapling device, comprising:
an end effector including an anvil jaw member and a cartridge jaw member pivotally coupled to one another, the anvil jaw member and the cartridge jaw member being relatively movable such that the end effector is movable between an open position and a clamped position;
a first buttress attached to the anvil jaw member, the first buttress having at least one therapeutic agent thereon; and
a second buttress attached to the cartridge jaw member.

2. The surgical stapling device of claim 1, wherein the second buttress has at least one therapeutic agent thereon.

3. The surgical stapling device of claim 1 or claim 2, wherein the therapeutic agent is any combination of amino acids, peptides, polypeptides, proteins, polysaccharides, muteins, immunoglobulins, antibodies, cytokines, blood clotting factors, hemopoietic factors, interleukins (1 through 18), interferons, erythropoietin, nucleases, tumor necrosis factor, colony stimulating factors, insulin, anti-tumor agents and tumor suppressors, blood proteins, fibrin, thrombin, fibrinogen, synthetic thrombin, synthetic fibrin, synthetic fibrinogen, gonadotropins, hormones and hormone analogs, vaccines, somatostatin, antigens, blood coagulation factors, growth factors, bone morphogenic proteins, TGF-B, protein inhibitors, protein antagonists, protein agonists, nucleic acids, such as antisense molecules, DNA, RNA, RNAi, oligonucleotides, polynucleotides, cells, viruses, anti-inflammatory agents, anti-bacterial agents, antimicrobial agents, and ribozymes.

4. The surgical stapling device of any preceding claim, wherein the therapeutic agent is a chemotherapy drug; preferably wherein the chemotherapy drug is any combination of paclitaxel and derivatives thereof, docetaxel and derivatives thereof, abraxane, tamoxifen, cyclophosphamide, actinomycin, bleomycin, dactinomycin, daunorubicin, doxorubicin, doxorubicin hydrochloride, epirubicin, mitomycin, methotrexate, fluorouracil, gemcitabine, gemcitabine hydrochloride, carboplatin, carmustine, methyl-CCNU, cisplatin, etoposide, camptothecin and derivatives thereof, phenesterine, vinblastine, vincristine, goserelin, leuprolide, interferon alfa, retinoic acid, nitrogen mustard alkylating agents, piposulfan, vinorelbine, irinotecan, irinotecan hydrochloride, vinblastine, pemetrexed, sorafenib tosylate, everolimus, erlotinib hydrochloride, sunitinib malate, capecitabine oxaliplatin, leucovorin calcium, bevacizumab, cetuximab, ramucirumab, trastuzumab, atezolizumab, canakinumab, and combinations thereof.

5. The surgical stapling device of any preceding claim, wherein the therapeutic agent is combined with an excipient including a surfactant, a salt, an acid, a stabilizer, a polyhydric alcohol, a hydrotrope, a low molecular weight poly(ethylene glycol) or any combination thereof; preferably wherein the surfactant is a cyclodextrin, sodium dodecyl sulfate, octyl glucoside, a sorbitan fatty acid ester, or combinations thereof.

6. The surgical stapling device of claim 5, wherein the salt includes sodium chloride; and/or wherein the acid includes oleic acid, citric acid, ascorbic acid, or combinations thereof; and/or wherein the stabilizer includes butylated hydroxytoluene, or butylated hydroxyanisole; and/or wherein the polyhydric alcohol includes D-sorbitol, mannitol, or combinations thereof.

7. The surgical stapling device of any preceding claim, wherein the first buttress is attached to the anvil jaw member by at least one suture.

8. The surgical stapling device of any preceding claim, wherein the second buttress is attached to the cartridge jaw member by at least one suture.

9. A surgical stapling device, comprising:
an end effector including an anvil jaw member and a cartridge jaw member pivotally coupled to one another, the anvil jaw member and the cartridge jaw member being relatively movable such that the end effector is movable between an open position and a clamped position;
a first buttress attached to the anvil jaw member, the first buttress having at least one chemotherapy drug thereon; and
a second buttress attached to the cartridge jaw member.

10. The surgical stapling device of claim 9, wherein the second buttress has at least one chemotherapy drug thereon.

11. The surgical stapling device of claim 9 or claim 10, wherein the chemotherapy drug is any combination of paclitaxel and derivatives thereof, docetaxel and derivatives thereof, abraxane, tamoxifen, cyclophosphamide, actinomycin, bleomycin, dactinomycin, daunorubicin, doxorubicin, doxorubicin hydrochloride, epirubicin, mitomycin, methotrexate, fluorouracil, gemcitabine, gemcitabine hydrochloride, carboplatin, carmustine, methyl-CCNU, cisplatin, etoposide, camptothecin and derivatives thereof, phenesterine, vinblastine, vincristine, goserelin, leuprolide, interferon alfa, retinoic acid, nitrogen mustard alkylating agents, piposulfan, vinorelbine, irinotecan, irinotecan hydrochloride, vinblastine, pemetrexed, sorafenib tosylate, everolimus, erlotinib hydrochloride, sunitinib malate, capecitabine oxaliplatin, leucovorin calcium, bevacizumab, cetuximab, ramucirumab, trastuzumab, atezolizumab, canakinumab, and combinations thereof.

12. The surgical stapling device of claim 10 or claim 11, wherein the chemotherapy drug is combined with an excipient including a surfactant, a salt, an acid, a stabilizer, a polyhydric alcohol, a hydrotrope, a low molecular weight poly(ethylene glycol) or any combination thereof.

13. The surgical stapling device of any of claims 9 to 12, wherein the first buttress is attached to the anvil jaw member by at least one suture; and/or wherein the second buttress is attached to the cartridge jaw member by at least one suture.
